# EUROPÄISCHE PATENTANMELDUNG

(11) **EP 1 579 861 A2**
(43) Veröffentlichungstag der Anmeldung: **28.09.2005**
(21) Anmeldenummer: 05006483.1
(22) Anmeldetag: 24.03.2005
(51) Int. Cl.: A61K 31/4166, A61K 31/164, A61P 17/00, A61P 31/00

(54) **Salbe zur Pflege des Nagelbettes und zur Behandlung von oder Prophylaxe vor Nagelbettentzündung und/oder Entzündungen der Nasenschleimhäute**

(30) Priorität: 26.03.2004 DE 102004020890
(71) Anmelder: Wolf, Wilhelm R., 41515 Grevenbroich (DE)
(72) Erfinder: Wolf, Wilhelm R., 41515 Grevenbroich (DE)
(74) Vertreter: Jabbusch, Wolfgang

(57) **Zusammenfassung**

Die vorliegende Erfindung betrifft die Verwendung von Allantoin oder von einem seiner pharmazeutische verträglichen Salze oder Derivate zur Herstellung eines Medikaments zur Behandlung von oder Prophylaxe vor Nagelbettentzündung oder Entzündungen im Bereich der Nase. In einer besonders bevorzugten Ausführungsform enthält das Medikament neben Allantoin zusätzlich D-Pantenol.

## Beschreibung

Die vorliegende Erfindung betrifft die Verwendung von Allantoin oder von einem seiner pharmazeutisch verträglichen Salze oder Derivate zur Herstellung eines Medikaments zur Pflege des Nagelbettes und/oder zur Behandlung von oder Prophylaxe vor Nagelbettentzündungen oder Entzündungen im Bereich der Nase.

Allantoin, chemisch (RS)-(2,5-Dioxo-4-imidazolidinyl)-harnstoff, auch als 5-Ureidohydantoin oder Glyoxylsäurediureid bezeichnet, ist in der Natur weit verbreitet. Bei den meisten Säugetieren ist Allantoin das Endprodukt des Purinstoffwechsels, wobei die Harnsäure durch das Enzym Uricase zu Allantoin abgebaut wird. Allantoin wird hauptsächlich im kosmetischen Bereich eingesetzt, wo es in vielen Hautcremes, Sonnenschutzmitteln und Rasierwässern enthalten ist. Im medizinischen Bereich wird es bisher in Wundsalben, insbesondere zur Behandlung von Schnitt- und Brandwunden, Geschwüren, Röntgenschäden und Ekzemen eingesetzt (siehe Hunnius, Pharmazeutisches Wörterbuch, 8. Auflage, Walter de Gruyter, Berlin, New York, 1998).

Der gegenwärtige Erfinder hat nun überraschend festgestellt, dass Allantoin bzw. eines seiner pharmazeutisch verträglichen Salze oder Derivate (in der folgenden Beschreibung soll der Begriff Allantoin so verstanden werden, dass er diese Salze und Derivate mit umfasst) hervorragend zur Behandlung von oder Prophylaxe vor Nagelbettentzündungen oder Entzündungen im Bereich der Nase eingesetzt werden kann. Allantoin kann außerdem auch zur Pflege des Nagelbettes eingesetzt werden.

Allantoin wird dabei gemäß der vorliegenden Erfindung vorzugsweise für die topische Verabreichung formuliert, insbesondere in Form einer Salbe, Lotion oder Creme. Daneben sind auch wässrige Lösungen, Suspensionen oder Emulsionen oder weitere Formulierungen, die auf die Haut aufgetragen werden können, einsetzbar. Allantoin wird bevorzugt in einer Menge eingesetzt, die von 0,2 bis 20 Gew.-%, vorzugsweise 0,5 bis 10 Gew.-%, insbesondere 1 bis 5 Gew.-% der Formulierung reicht. Eine besonders bevorzugte Ausführungsform enthält ca. 2 Gew.-% des aktiven Bestandteils.

Als pharmazeutisch verträgliche Salze oder Derivate kommen sämtliche chemisch möglichen und zur Anwendung am Menschen zulässigen Verbindungen in Frage, die in einen pharmazeutischen Träger eingearbeitet werden können und therapeutisch wirksam sind.

Als pharmazeutische Träger kommen ebenfalls alle verfügbaren Trägermaterialien in Betracht. Für topische Formulierungen eignen sich hierfür alle Salben-, Creme- und Lotionzubereitungen und -grundlagen.

Die Salbe, Lotion oder Creme stellt sowohl von der Viskosität als auch von der Oberflächenspannung eine längere Benetzung des betroffenen Bereichs und damit einen intensiven Kontakt der Wirksubstanz/en mit der Haut sicher.

Mögliche Bestandteile dieser Formulierungen sind beispielsweise Wasser, pflanzliche Öle, Polyalkylenglykole, Vaseline, Lanolin, Paraffinöl sowie alle für topische Formulierungen einsetzbaren Komponenten.

Die Formulierungen können darüber hinaus Hilfsstoffe wie Konservierungsmittel, Stabilisatoren, Antioxidantien, Verdickungsmittel, Duftstoffe, Emulgatoren, Puffersubstanzen, Farbstoffe, Pigmente usw. enthalten.

Der gegenwärtige Erfinder hat ebenfalls festgestellt, dass die Wirkung von Allantoin verbessert werden kann, wenn dieses zusammen mit D-Pantenol oder einem seiner pharmazeutisch verträglichen Salze oder Derivate verwendet wird. D-Pantenol, ebenfalls bekannt als Dexpantenol, chemisch D-(+)-2,4-Dihydroxy-N-(3-hydroxypropyl)-3,3-dimethylbutyramid, wird im Organismus zu Pantothensäure oxidiert und ist als Bestandteil von Wundsalben bekannt (z.B. Bepanthen® Roche Wund- und Heilsalbe).

Wenn neben Allantoin als wirksamem Bestandteil zusätzlich D-Pantenol oder eines seiner pharmazeutisch verträglichen Salze oder Derivate in die Formulierung eingearbeitet wird, ist dieses vorzugsweise in einer Menge von 0,2 bis 20 Gew.-%, vorzugsweise 1 bis 15 Gew.-%, insbesondere 2 bis 10 Gew.-% enthalten. Eine besonders bevorzugte Ausführungsform enthält ca. 5 Gew.-% des aktiven Bestandteils.

Auch weitere wirksame Bestandteile, z.B. zusätzliche bekannte entzündungshemmende und/oder hautentspannende und/oder pflegende Komponenten, können neben Allantoin und gegebenenfalls D-Pantenol in die Formulierungen eingearbeitet werden. Beispiele hierfür sind Vitamin E, Aloe Vera, Kamilleextrakte und weitere Substanzen, die üblicherweise im kosmetischen Bereich eingesetzt werden. Diese Substanzen können sowohl natürlichen als auch synthetischen Ursprungs sein.

Die topischen Formulierungen können hergestellt werden, indem die oben genannten aktiven Bestandteile mit dem gewünschten pharmazeutischen Träger gemischt werden. Hierzu können allgemein bekannte Mischtechniken eingesetzt werden, wie sie z.B. in der pharmazeutischen oder kosmetischen Industrie sowohl labor- als auch großtechnisch angewandt werden um Bestandteile gleicher und unterschiedlicher Löslichkeit mit und ohne Zusatz von Lösungsvermittlern oder anderen Zusatzstoffen zu mischen und/oder stabil zu emulgieren oder thixotrop zu stabilisieren. Die Mischtechniken können manuell oder maschinell mit oder ohne Wärmezufuhr ein- oder mehrphasige Lösungs-, Extraktions-, Homogenisierungsprozesse mit und ohne Nutzung von partiellen Mischungslücken und Entmischungen beinhalten.

Die jeweilige Mischtechnik wird bestimmt durch Variable wie z.B. aber nicht nur Viskosität, Lösungsverhalten und Verhältnis von wasser- zu fettlöslichen Bestandteilen. Die Formulierungen können in zwei oder mehr Chargen vorgemischt und in der Endformulierung zum eigentlichen Produkt abgemischt werden. Diese Vorgehensweise kann in Abhängigkeit von den einzelnen Bestandteilen der Formulierung modifiziert werden, insbesondere wenn wasserfreie Formulierungen Anwendung finden.

Die erhaltenen Präparate können dann je nach Bedarf einmal oder mehrmals täglich auf den betroffenen Bereich des Patienten aufgetragen werden. Zur Prophylaxe kann eine Auftragung beispielsweise ein bis dreimal täglich im Abstand von jeweils ca. vier Stunden erfolgen. Für eine Behandlung wird vorzugsweise eine Auftragung drei- bis fünfmal täglich für zwei bis fünf Tage, ggf. auch länger, eingesetzt. In der Regel empfiehlt es sich, die Behandlung nach Abklingen der Entzündung noch einen weiteren Tag fortzusetzen.

Grundsätzlich muss der Arzt unter Berücksichtigung der Gesamtumstände über Umfang und Häufigkeit der Anwendung sowie die Kombination mit anderen Therapiemaßnahmen, insbesondere in schweren Fällen, entscheiden. Sollten keine anderen Empfehlungen gegeben werden oder ist auf Grund besonderer Umstände eine Konsultation nicht möglich oder besteht in besonders leichten Fällen ohne erschwerenden Verlauf gegen eine Selbstmedikation kein Einwand, so ist die in den unten folgenden Beispielen zur Prophylaxe beschriebene Behandlung empfehlenswert.

Als mögliche Salbengrundlagen sind insbesondere die folgenden geeignet:
A) weiße Vaseline
   wasserfreies Lanolin
   Paraffinöl
   Ethyl-4-hydroxybenzoat
   destilliertes Wasser
B) weiße Vaseline
   wasserfreies Lanolin
   Paraffinöl
   Propylenglykol
   Ethyl-4-hydroxybenzoat
   destilliertes Wasser
C) wasserfreies Lanolin
   Paraffinöl
   Mischung aus Cetyl- und Stearylalkohol
   Polyoxyethylensorbitanmonostearat
   Propylenglykol
   Ethyl-4-hydroxybenzoat
   Methyl-4-hydroxybenzoat
   Propyl-4-hydroxybenzoat
   destilliertes Wasser

Eine besonders bevorzugte Anwendung neben der Behandlung von Nagelbettentzündungen besteht in der Prophylaxe vor Nagelbettentzündungen bei Verletzungen des Nagelbetts (Hand oder Fuß) bei Mensch und Tier. Eine prophylaktische Anwendung kommt ebenfalls bei der Entfernung von Beinägeln, einem zu kurzen Anschnitt bei der Pediküre oder Maniküre usw. in Betracht.

Bei der Anwendung von Allantoin oder Allantoin plus D-Pantenol wurde überraschend festgestellt, dass praktisch unmittelbar nach der Auftragung einer Allantoin und ggf. D-Pantenol enthaltenden Formulierung eine schmerzstillende Wirkung auftrat. Dabei wurde insbesondere auch eine schmerzstillende Wirkung bei dem oft als sehr unangenehm empfundenen Berührungsschmerz bzw. eine erhebliche Minderung der Berührungsempfindlichkeit festgestellt.

Neben der Wirksamkeit von Allantoin zur Behandlung von oder Prophylaxe vor Nagelbettentzündungen hat der gegenwärtige Erfinder festgestellt, dass ebenfalls Entzündungen der Naseninnenwände bzw. der Nasenschleimhäute, aber auch der Nasenaußenwand, erfolgreich mit Allantoin oder einer Kombination aus Allantoin und D-Pantenol behandelt werden können oder prophylaktisch verhindert werden können. Hierzu können dieselben Formulierungen, wie sie oben zur Verwendung bei Nagelbettentzündung beschrieben wurden, eingesetzt werden.

Als Applikatoren bei einer Anwendung im Nasenraum eignen sich dabei aller Behältnisse mit einer Austrittsöffnung, die einen dünnen Strang erzeugt, und die weit genug in den Nasenraum eingebracht werden können.

Die oben beschriebenen Formulierungen bzw. Anwendungen sind einfach zu lagern bzw. anzuwenden und werden in der Regel vom Anwender problemlos vertragen. Die Anwendungen sind ebenfalls zusammen mit anderen Behandlungen möglich.

Im Folgenden wird die vorliegende Erfindung anhand von spezifischen Ausführungsbeispielen noch einmal näher erläutert. Diese Ausführungsbeispiele sollen die vorliegende Erfindung jedoch nicht beschränken, so dass Abwandlungen hiervon immer noch unter den Umfang der Erfindung fallen.

### Beispiel 1

### Herstellung einer Allantoin-haltigen Salbe

In 49 Gramm einer Salbengrundlage der oben beschriebenen Zusammensetzung A) wurde unter Rühren 1 Gramm (2 Gew.-%) Allantoin eingearbeitet, bis sich eine homogene Mischung ergab. Dabei wird das Allantoin bevorzugt durch vorhergehende Lösung in einem geeigneten Material, das Bestandteil der Salbengrundlage ist, vorbereitet.

Anwendung der hergestellten Salbe zur Behandlung von Nagelbettentzündung Ein dünner Strang der so erhaltenen Allantoin-haltigen Salbe wurde bei einem Patienten mit Nagelbettentzündung am großen Zeh des linken Fußes seitlich möglichst dicht an der betroffenen Stelle aufgebracht und leicht einmassiert. Überschüssige Salbe wurde an der betroffenen Stelle belassen und mit Verbandsmaterial abgedeckt, um eine längere Einwirkzeit zu erzielen. Die Behandlung wurde dreimal täglich für fünf Tage durchgeführt.

Schon nach dem ersten Auftragen der Salbe stellte der Patient eine deutliche schmerzstillende Wirkung fest. Der Berührungsschmerz ließ unmittelbar nach. Nach drei Tagen war die Entzündung deutlich zurückgegangen und nach fünf Tagen war keine Entzündung mehr feststellbar.

### Beispiel 2

### Herstellung einer Salbe, die Allantoin und D-Pantenol enthielt

In 27,9 Gramm einer Salbengrundlage der oben beschriebenen Zusammensetzung B) wurden unter Rühren 0,6 Gramm Allantoin (2 Gew.-%) und 1,5 Gramm D-Pantenol (5 Gew.-%) eingearbeitet, bis sich eine homogene Mischung ergab.

### Anwendung der hergestellten Salbe zur Behandlung von Nagelbettentzündung

Die so erhaltene Allantoin und D-Pantenol enthaltende Salbe wurde bei einer Patientin mit Nagelbettentzündung am rechten Ringfinger dreimal täglich unmittelbar auf das betroffene Nagelbett aufgetragen.

Schon nach dem ersten Auftragen der Salbe stellte die Patientin eine deutliche Abnahme des Schmerzes fest. Nach zwei Tagen war ein Rückgang der Entzündung feststellbar und nach vier Tagen war die Nagelbettentzündung vollkommen abgeklungen.

### Beispiel 3

### Anwendung der hergestellten Salbe zur Prophylaxe vor Nagelbettentzündungen bei Verletzung des Nagelbetts

Die in Beispiel 1 hergestellte Salbe wurde wie folgt zur Prophylaxe vor Nagelbettentzündungen eingesetzt: Bei einem Patienten mit einer Verletzung am Nagelbett des kleinen Fingers der rechten Hand wurde dreimal im Abstand von jeweils vier Stunden ein dünner Strang der Salbe seitlich möglichst dicht an dem betroffenen Bereich aufgetragen und einmassiert. Überschüssiges Material wurde an dem betroffenen Bereich belassen und mit einer Mullbinde abgedeckt.

Der Patient entwickelte keine Nagelbettentzündung, und die Verletzung heilte problemlos.

### Beispiel 4

### Anwendung der hergestellten Salbe zur Behandlung von Entzündungen der Nasenscheidewand

Ein dünner Strang der in Beispiel 2 hergestellten Salbe wurde möglichst dicht an der betroffenen Stelle der Nasenscheidewand eines betroffenen Patienten aufgebracht und leicht einmassiert. Überschüssiges Material wurde an der Stelle belassen, soweit es die Atmung nicht beeinträchtigte, um eine längere Einwirkzeit zu erzielen. Die Behandlung wurde viermal täglich für drei Tage durchgeführt.

Schon nach dem ersten Auftragen der Salbe stellte der Patient eine deutliche Abnahme des Schmerzes fest. Nach drei Tagen war die Entzündung vollkommen abgeklungen.

### Beispiel 5

### Anwendung der hergestellten Salbe zur Prophylaxe vor einer Entzündung der Nasenaußenwand

Die in Beispiel 1 hergestellte Salbe wurde wie folgt zur Prophylaxe vor einer Entzündung der Nasenaußenwand eingesetzt: Bei einer Patientin mit einer Verletzung am rechten äußeren Nasenflügel wurde dreimal im Abstand von jeweils vier Stunden ein dünner Strang der Salbe auf den betroffenen Bereich aufgetragen und einmassiert. Überschüssiges Material wurde an dem betroffenen Bereich belassen und mit einem Pflaster abgedeckt.

Die Patientin entwickelte keine Entzündung, und die Verletzung heilte problemlos.

## Patentansprüche

1. Verwendung von Allantoin oder seinen pharmazeutisch verträglichen Salzen oder Derivaten zur Herstellung eines Medikaments zur Pflege des Nagelbettes und/oder zur Behandlung von oder Prophylaxe vor Nagelbettentzündungen.

2. Verwendung nach Anspruch 1, **dadurch gekennzeichnet, dass** der aktive Bestandteil in Kombination mit einem pharmazeutisch verträglichen Träger verwendet wird.

3. Verwendung nach Anspruch 1 oder 2, **dadurch gekennzeichnet, dass** das Medikament für die topische Verabreichung formuliert wird.

4. Verwendung nach einem der Ansprüche 1 bis 3, **dadurch gekennzeichnet, dass** das Medikament als Salbe, Lotion oder Creme formuliert wird.

5. Verwendung nach einem der Ansprüche 1 bis 4, **dadurch gekennzeichnet, dass** das Medikament 0,2 bis 20 Gew.-%, vorzugsweise 0,5 bis 10 Gew.-%, insbesondere 1 bis 5 Gew.-% und besonders bevorzugt ca. 2 Gew.-% des aktiven Bestandteils enthält.

6. Verwendung nach einem der Ansprüche 1 bis 5, **dadurch gekennzeichnet, dass** das Medikament zusätzlich D-Pantenol oder pharmazeutisch verträgliche Salze oder Derivate von diesem als weiteren aktiven Bestandteil enthält.

7. Verwendung nach Anspruch 6, **dadurch gekennzeichnet, dass** D-Pantenol in dem Medikament in einer Menge von 0,2 bis 20 Gew.-%, vorzugsweise 1 bis 15 Gew.-%, insbesondere 2 bis 10 Gew.-% und besonders bevorzugt ca. 5 Gew.-% enthalten ist.

8. Verwendung von Allantoin oder seinen pharmazeutisch verträglichen Salzen oder Derivaten zur Herstellung eines Medikaments zur Behandlung von oder Prophylaxe vor Entzündungen im Bereich der Nase.

9. Verwendung nach Anspruch 8, **dadurch gekennzeichnet, dass** das Medikament zusätzlich D-Pantenol oder pharmazeutisch verträgliche Salze oder Derivate von diesem als weiteren aktiven Bestandteil enthält.

10. Verwendung nach Anspruch 8 oder 9, **dadurch gekennzeichnet, dass** es sich bei den Entzündungen im Bereich der Nase um Entzündungen der Naseninnenwände bzw. der Nasenschleimhaut oder um Entzündungen der Nasenaußenwände handelt.
